# EUROPÄISCHE PATENTANMELDUNG

(11) **EP 2 762 292 A1**
(43) Veröffentlichungstag der Anmeldung: **06.08.2014**
(21) Anmeldenummer: 14166851.7
(22) Anmeldetag: 25.06.2009
(51) Int. Cl.: B29C 49/42, A61L 2/08, B65B 55/08, B67C 3/26, B29C 49/64

(54) **Vorrichtung und Verfahren zum Herstellen von Kunststoffbehältnissen**

(30) Priorität: 27.06.2008 DE 102008030156
(62) Teilanmeldung aus: 09163763.7
(71) Anmelder: Krones AG, 93073 Neutraubling (DE)
(72) Erfinder: Krüger, Jochen, 93073 Neutraubling (DE); Faltermeier, Manfred, 93073 Neutraubling (DE)
(74) Vertreter: Hannke, Christian

(57) **Zusammenfassung**

Eine Vorrichtung (1) zum Herstellen von Kunststoffbehältern (20) mit einer Umformungseinrichtung (6), welche Kunststoffvorformlinge (10) zu Kunststoffbehältnissen (20) umformt, mit einer ersten Transporteinrichtung (2), welche die Kunststoffvorformlinge (10) der Umformungseinrichtung (6) zuführt und einer zweiten Transporteinrichtung (12), welche die Kunststoffbehältnisse (20) von der Umformungseinrichtung abführt. Erfindungsgemäß weist die Vorrichtung eine erste Sterilisationseinrichtung (4) zum Sterilisieren der Kunststoffvorformlinge (10), welche in einer Transportrichtung der Kunststoffvorformlinge vor der Umformungseinrichtung (6) angeordnet ist und eine zweite Sterilisationseinrichtung (8) zum Sterilisieren der Kunststoffbehältnisse (20), welche in der Transportrichtung der Kunststoffbehältnisse (20) nach der Umformungseinrichtung (6) angeordnet ist.

## Beschreibung

Die Erfindung bezieht sich auf eine Vorrichtung und ein Verfahren zum Herstellen von Kunststoffbehältnissen und insbesondere von Kunststoffbehältnissen für Getränke. Es wird jedoch auch darauf hingewiesen, dass die hergestellten Behältnisse auch für andere Flüssigkeiten verwendbar sind. Aus dem Stand der Technik ist eine Vielzahl von Vorrichtungen zum Herstellen von Kunststoffbehältnissen bekannt. Dabei ist es insbesondere bekannt, dass sogenannte Kunststoffvorformlinge einer Blaseinrichtung zugeführt werden wobei diese Blaseinrichtung mit Hilfe von Luftdruck die Kunststoffvorformlinge zu Kunststoffbehältnissen expandiert.

Im Stand der Technik tritt jedoch dabei des Öfteren das Problem auf, dass während des Expansionsvorgangs oder auch danach Verunreinigungen der Behältnisse auftreten können oder aber bereits die Vorformlinge verunreinigt sind. Aus diesem Grunde ist es im Stand der Technik bekannt, die erzeugten Behältnisse mit Hilfe von Chemikalien zu reinigen.

So ist beispielsweise aus der EP 0996530 eine chemische Preformentkeimung vor dem Einlauf in einen Heizofen bekannt oder auch das Injizieren von wärmeaktivierbarem Desinfektionsmittel in die Preform und ein anschließendes Erwärmen der Preform in einer Blasmaschine auf eine Aktivierungstemperatur des Desinfektionsmittels sowie eine nachfolgende Erwärmung auf eine Verformungstemperatur. Weiterhin offenbart die US 6,692,684 ein Verfahren, bei dem die Preforms zunächst über den Taupunkt des Desinfektionsmittels erwärmt werden und das Desinfektionsmittel gasförmig eingebracht wird und anschließend eine Erwärmung auf Umformungstemperatur erfolgt.

Die Verwendung derartiger Chemikalien ist jedoch sowohl aus umweltpolitischen Gründen als auch aus Kostengründen problematisch. Der vorliegenden Erfindung liegt daher die Aufgabe zugrunde, eine Vorrichtung und ein Verfahren zur Herstellung von Kunststoffbehältnissen zur Verfügung zu stellen, welches effizient arbeitet und gleichzeitig den Einsatz von Chemikalien zumindest reduziert.

Dies wird erfindungsgemäß durch eine Vorrichtung zum Herstellen von Kunststoffbehältnissen nach Anspruch 1 und ein Verfahren zum Herstellen von Kunststoffbehältnissen nach Anspruch 12 erreicht. Vorteilhafte Ausführungsformen und Weiterbildungen sind Gegenstand der Unteransprüche.

Eine erfindungsgemäße Vorrichtung zum Herstellen von Kunststoffbehältnissen weist eine Umformungseinrichtung auf, welche Kunststoffvorformlinge zu Kunststoffbehältnissen umformt sowie eine erste Transporteinrichtung, welche die Kunststoffvorformlinge der Umformungseinrichtung zuführt und eine zweite Transporteinrichtung, welche die Kunststoffbehältnisse von der Umformungseinrichtung abführt. Erfindungsgemäß weist die Vorrichtung eine erste Sterilisationseinrichtung zum Sterilisieren der Kunststoffvorformlinge auf, welche in einer Transportrichtung der Kunststoffvorformlinge vor der Umformungseinrichtung angeordnet ist sowie eine zweite Sterilisationseinrichtung zum Sterilisieren der Kunststoffbehältnisse, welche in einer Transportrichtung der Kunststoffbehältnisse nach der Umformungseinrichtung angeordnet ist.

Damit wird erfindungsgemäß vorgeschlagen, dass eine zweifache Sterilisation vorgenommen wird nämlich einerseits der Kunststoffvorformlinge vor dem Umformungsvorgang und andererseits auch der nach dem Umformungsvorgang entstandenen Kunststoffbehältnisse. Auf diese Weise kann eine effiziente Sterilisation erreicht werden, ohne hierzu in erhöhtem Maße auf chemische Reinigungsmittel zurückgreifen zu müssen. Gleichwohl ist es auch denkbar, dass wenigstens einer der Sterilisationsvorgänge oder auch ein zusätzlicher Sterilisationsvorgang auch durch die Verwendung von Chemikalien und insbesondere von Wasserstoffperoxid (H₂O₂) gestützt wird.

Bei einer bevorzugten Ausführungsform weist die erste Sterilisationseinrichtung eine Strahlungsquelle auf, mittels derer die Kunststoffvorformlinge mit einer Strahlung und insbesondere einer elektromagnetischen Strahlung beaufschlagt werden. Dabei kann es sich um eine Strahlungsquelle handeln, welche die Behältnisse mit einer UV-Strahlung beaufschlagt, bevorzugt wird jedoch eine Strahlungsquelle verwendet, welche Elektronenstrahlen auf die Behältnisse richtet. Es wäre jedoch auch möglich, dass die Strahlungsquelle andere Strahlungsarten wie beispielsweise Laserstrahlung auf die Behältnisse richtet. Damit kann bevorzugt im Rahmen der Sterilisation durch die erste Sterilisationseinrichtung auf den Einsatz von Chemikalien verzichtet werden.

Bei einer weiteren vorteilhaften Ausführungsform weist die zweite Sterilisationseinrichtung eine Strahlungsquelle auf, mittels derer die Kunststoffbehältnisse mit einer Strahlung beaufschlagt werden. Bevorzugt kann es sich auch hier um eine Strahlungsquelle handeln, welche Elektronenstrahlung, Laserstrahlung, UV-Strahlung, Kombinationen hieraus oder dergleichen abgibt. Besonders bevorzugt beaufschlagt die erste Sterilisationseinrichtung die Kunststoffvorformlinge mit Elektronenstrahlen wohingegen die zweite Sterilisationseinrichtung die Kunststoffbehältnisse mit UV-Strahlung beaufschlagt. Damit findet durch die zweite Sterilisationseinrichtung insbesondere eine Nachsterilisation der bereits durch die erste Sterilisationseinrichtung vorsterilisierten Behältnisse statt. Bevorzugt dient die zweite Sterilisationseinrichtung insbesondere dazu, um nach der ersten Sterilisation aufgetretene Verkeimungen zu beseitigen.

Durch den Blasprozess und den weiteren Transport verursachte Verkeimungen werden dadurch beseitigt, dass, bevorzugt in einer Transfertransporteinrichtung zwischen der Umformungseinheit und beispielsweise einer Befüllungseinrichtung, eine weitere Sterilisationseinrichtung vorgesehen ist, die ebenso bevorzugt keine chemischen Mittel einsetzt. Dazu werden die Kunststoffbehältnisse bevorzugt mittels eines Lineartransfers mit UV - Lampen bestrahlt. Dabei ist es möglich, dass die Kunststoffbehältnisse in ihren Halteeinrichtungen beispielsweise sog. Neckhandlingklammern gedreht werden, damit durch das Neckhandling keine Schatten entstehen, die eine sichere Entkeimung verhindern.

Es wird darauf hingewiesen, dass es auch möglich ist, die erste Sterilisationseinrichtung in einem Bereich der Umformungseinrichtung und insbesondere in einem Einlaufbereich derselben anzuordnen. Auch wäre es denkbar, dass die Sterilisation zumindest zeitweise zeitgleich mit dem Expansionsvorgang stattfindet. Daneben könnte auch die zweite Sterilisationseinrichtung in einem Bereich der Umformungseinrichtung und/oder auch in einem Bereich eines Auslaufs derselben vorgesehen sein.

Auch wäre es möglich, dass in den einzelnen Blasstationen der Umformungseinrichtung Sterilisationseinrichtungen angeordnet sind. So könnten beispielsweise an den Innenwandungen von Blasformen, welche den zu expandierenden Behältnissen zugewandt sind, Strahlungsquellen wie UV-Lampen, angeordnet sein, welche die Behältnisse während des Expansionsvorgangs bestrahlen. Auch wäre es möglich, insbesondere UV - Strahlungsquellen derart anzuordnen, dass die Kunststoffvorformlinge während des Schließens einer Blasform oder die Kunststoffbehältnisse während des Öffnens einer Blasform mit UV - Strahlung beaufschlagt werden.

Daneben wäre es auch möglich, während des Expansionsvorgangs, beispielsweise über eine Reckstange, ein Sterilisationsmedium, wie beispielsweise Wasserstoffperoxid in die Behältnisse einzuführen

Die oben beschriebene Sterilisation der Vorformlinge bietet den Vorteil, dass die Oberflächen dieser Vorformlinge kleiner sind als diejenige der bereits fertig geblasenen Behältnisse und daher der Aufwand, insbesondere beim Einsatz von Elektronenstrahlen geringer ist.

Bei einer weiteren bevorzugten Ausführungsform weist die Vorrichtung eine Dreheinrichtung auf, welche die Vorformlinge während der Sterilisation durch die erste und/oder die zweite Sterilisationseinrichtung dreht. Damit ist es möglich, dass eine Strahlungsquelle drehfest angeordnet ist und die Vorformlinge diese gegenüber gedreht werden, um einen größeren Bereich der Vorformlinge zu sterilisieren. Dabei führt die erste Sterilisationseinrichtung insbesondere auch einen Sterilisation der Innenwandung der Vorformlinge durch. Es wäre jedoch auch möglich, dass vor der Umformungseinrichtung mehrere Sterilisationseinrichtungen vorgesehen sind, welche die Behältnisse sowohl an ihrer Außenwandung als auch an ihrer Innenwandung und insbesondere mit Elektronenstrahlen beaufschlagen.

Bevorzugt ist eine Vielzahl derartiger Dreheinrichtungen vorgesehen, wobei bevorzugt die Dreheinrichtungen an Transportelementen, wie beispielsweise Greifklammern vorgesehen sind, so dass mehrere Vorformlinge gleichzeitig transportiert und gedreht werden können. Bei einer weiteren vorteilhaften Ausführungsform weist wenigstens eine Sterilisationseinrichtung und insbesondere die erste Sterilisationseinrichtung wenigstens eine Strahlungsquelle auf, deren Bewegung an die Bewegung der Kunststoffvorformlinge oder die Bewegung der Kunststoffbehältnisse gekoppelt ist.

Dies bedeutet, dass diese Strahlungsquelle mit den Kunststoffbehältnissen mitgeführt wird. Vorzugsweise findet diese Koppelung bei der ersten Sterilisationsvorrichtung statt. Es wäre jedoch umgekehrt auch möglich, dass die Behältnisse nicht gedreht werden und die besagte Strahlungsquelle der Sterilisationseinrichtung gedreht wird. Auch ist es möglich, dass die Strahlungsquellen stationär angeordnet sind und die Kunststoffvorformlinge sich gegenüber diesen stationär angeordneten Strahlungsquellen bewegen um auf diese Weise ebenfalls eine möglichst umfangreiche Sterilisation der Kunststoffvorformlinge zu erreichen. Weiterhin wäre es auch möglich, dass die Strahlungsquelle in einer Längsrichtung der Kunststoffvorformlinge diesen gegenüber bewegbar ist.

Dabei werden die Kunststoffvorformlinge mit Elektronenstrahlung (E-Beam) entkeimt. Dabei ist es möglich, insbesondere mit geringer Dosis von einer Mündung der Behältnisse her zu entkeimen, es wäre jedoch auch möglich, durch eine Seitenwand der Vorformlinge hindurch zu entkeimen, wobei hierzu bevorzugt hohe Beschleunigungsspannungen verwendet werden, um eine Durchdringung des Materials zu erhalten. Dabei kann, wie erwähnt, die Bestrahlung nach der Sortierung, im Einlaufrad der Heizeinrichtung, in der Heizeinrichtung oder im Transfer zwischen der Heizeinrichtung und der Umformungseinrichtung oder auch in einem Auslaufstern der Umformungseinrichtung bzw. Blasmaschine, oder auch an mehreren oder allen dieser Positionen erfolgen.

Bei einer weiteren vorteilhaften Ausführungsform ist wenigstens ein Abschnitt wenigstens einer Strahlungsquelle in die Mündung der zu sterilisierenden Kunststoffvorformlinge einführbar. So ist es möglich, dass die Strahlungsquelle ein Austrittsfenster aufweist, durch welches hindurch Elektronen hindurchtreten können. Diese Mündung kann dabei in einem Kopf angeordnet sein, der so dimensioniert ist, dass er durch die Mündung in den Innenraum des Kunststoffvorformlings einführbar ist.

Bei einer weiteren vorteilhaften Ausführungsform weist die Vorrichtung eine Heizeinrichtung auf, welche die Kunststoffvorformlinge erwärmt. Die so erwärmten Kunststoffvorformlinge können anschließend in der Umformungseinrichtung aufgrund der erhöhten Temperatur leichter zu Behältnissen umgeformt werden wobei die Umformungseinrichtung hierzu bevorzugt Düsen verwendet, welche die Kunststoffvorformlinge mit Luft beaufschlagen um diese so zu expandieren.

Vorzugsweise ist die erste Sterilisationseinrichtung in der Transportrichtung der Kunststoffvorformlinge vor der Heizeinrichtung angeordnet. Es wäre jedoch auch möglich, dass die erste Sterilisationseinrichtung innerhalb der Heizeinrichtung angeordnet ist oder unmittelbar nach der Heizeinrichtung und vor der ersten Umformungseinrichtung. Es könnten auch mehrere Sterilisationseinrichtungen vor der Umformungseinrichtung angeordnet sein, beispielsweise eine erste Sterilisationseinrichtung vor der Heizeinrichtung eine weitere in der Heizeinrichtung und eine weitere nach der Heizeinrichtung und vor der Umformungseinrichtung.

Bei einer weiteren vorteilhaften Ausführungsform ist die zweite Sterilisationseinrichtung in der Transportrichtung der Behältnisse unmittelbar nach der Umformungseinrichtung angeordnet. Auf diese Weise ist es möglich, dass die Behältnisse unmittelbar nach deren Erzeugen ein zweites Mal sterilisiert werden. Unter einer unmittelbaren Anordnung wird dabei insbesondere auch verstanden, dass die Behältnisse direkt von der Umformungseinheit über eine Transporteinrichtung, wie einen Transportstern, an die zweite Sterilisationseinrichtung übergeben werden.

Bevorzugt ist die zweite Sterilisationseinrichtung ebenfalls stationär angeordnet und die Behältnisse werden dieser gegenüber transportiert. Bei einer weiteren vorteilhaften Ausführungsform sind die Umformungseinrichtung und die Sterilisationseinrichtung innerhalb eines Gehäuses angeordnet, wobei dieses Gehäuse in seinem Inneren einen Sterilraum ausbildet. Diese bevorzugte Ausführungsform eignet sich insbesondere für die Abfüllung von sensiblen Getränken. Vorzugsweise ist in einem Bereich der ersten Sterilisationseinrichtung innerhalb des besagten Gehäuses ein zweites Gehäuse vorgesehen, wobei innerhalb des zweiten Gehäuses die erste Sterilisationseinrichtung angeordnet ist.

Dabei ist es möglich, dass innerhalb des zweiten Gehäuses ein weiterer Sterilraum ausgebildet ist, der einen noch höheren Reinheitsgrad aufweist als der Sterilraum innerhalb des ersten Gehäuses. Auf diese Weise ist es möglich, nicht das gesamte in üblicher Weise relativ große Gehäuse als hochwirksamen Sterilraum auszubilden, was mit hohen Kosten einhergehen würde, sondern lediglich einen relativ kleinen Bereich, in dem die erste Sterilisationseinrichtung angeordnet ist.

Die vorliegende Erfindung ist weiterhin auf ein Verfahren zum Herstellen von Kunststoffbehältnissen gerichtet, wobei einer Umformungseinrichtung Kunststoffvorformlinge mittels einer ersten Transproteinrichtung zugeführt werden und aus den Kunststoffvorformlingen erzeugte Kunststoffbehältnisse mittels einer zweiten Transporteinrichtung von der Umformungseinrichtung abgeführt werden. Erfindungsgemäß werden die Kunststoffvorformlinge auf ihren Transportpfad vor der Umformungseinrichtung und die aus den Kunststoffvorformlingen erzeugten Kunststoffbehältnisse auf Ihren Transportpfad nach der Umformungseinrichtung sterilisiert.

Im Rahmen umfangreicher Messungen hat sich gezeigt, dass es zwar teilweise möglich wäre, lediglich eine Sterilisationseinrichtung vor oder nach der Umformungseinrichtung vorzusehen, dass jedoch eine besonders kostengünstige und effiziente Vorgehensweise darin besteht, zwei Sterilisationseinrichtungen vorzugsehen, wobei eine Sterilisationseinrichtung vor der Umformungseinrichtung und die zweite Sterilisationseinrichtung nach der Umformungseinrichtung angeordnet ist.

Bei einem bevorzugten Verfahren erfolgt wenigstens die erste Sterilisation oder die zweite Sterilisation mittels Strahlung. Bevorzugt erfolgen die erste Sterilisation mittels Elektronenbestrahlung und die zweite Sterilisation mittels UV-Strahlung.

Weitere vorteilhafte Ausführungsformen ergeben sich aus den beigefügten Zeichnungen:
Darin zeigen:
   - Fig. 1: eine schematische Darstellung einer erfindungsgemäßen Vorrichtung;
   - Fig. 2: eine schematische Darstellung eines Bereichs der Vorrichtung aus Fig. 1, in dem die erste Sterilisationseinrichtung angeordnet ist; und
   - Fig. 3: eine schematische Darstellung einer Halterung für einen Kunststoffvorformling.

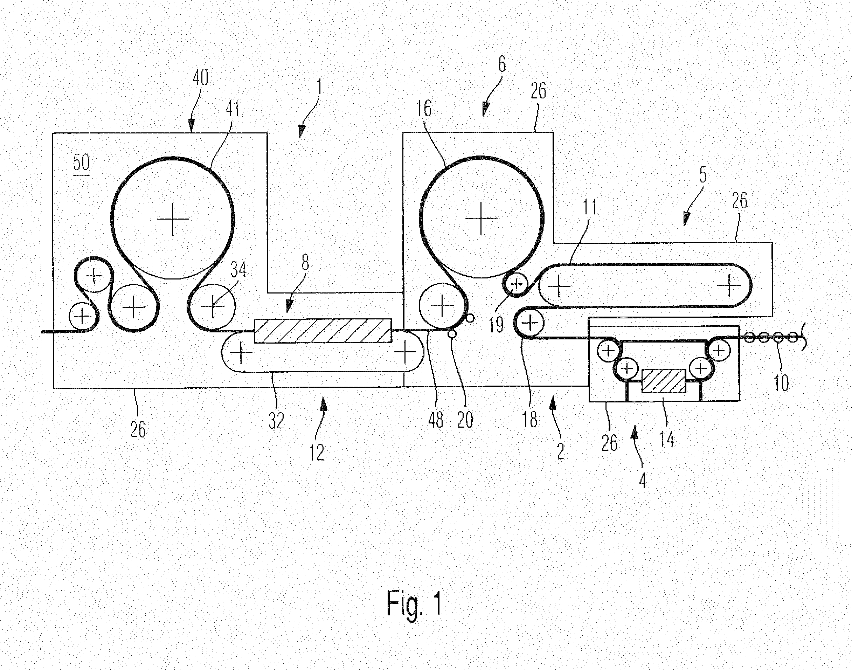

Fig. 1 zeigt eine erfindungsgemäße Vorrichtung 1 zum Herstellen von Behältnissen. Dabei werden Vorformlinge 10 über eine in ihrer Gesamtheit mit 2 bezeichnete Transporteinrichtung zu einer Umformungseinrichtung 6 geführt. Diese Umformungseinrichtung 6 erzeugt aus den Vorformlingen 10 Behältnisse 20, welche anschließend weiter in Richtung einer Befüllungseinrichtung 40 geführt werden. Die Transporteinrichtung 2 setzt sich dabei aus mehreren Transportmitteln zusammen wie beispielsweise einer Transportkette 11, einem Transportstern oder Transportrad 18 und einem weiteren Transportrad 19. Damit werden hier die Vorformlinge 10 bzw. später die Behältnisse 20 im Wesentlichen in Fig. 1 von rechts nach links gefördert.

Die Umformungseinrichtung 6 weist ein Blasrad 16 auf, an dem eine Vielzahl von Blasstationen angeordnet ist, welche die Vorformlinge 10 zu Behältnissen 20 in an sich aus dem Stand der Technik bekannter Weise, expandieren.

Das Bezugszeichen 5 bezieht sich auf eine Heizeinrichtung bzw. einen Ofen innerhalb dessen die Vorformlinge 10 erwärmt werden um anschließend die erwärmten Vorformlinge der Umformungseinrichtung 6 zuzuführen.

Vor dieser Heizeinrichtung 5 ist eine erste Sterilisationseinrichtung 4 angeordnet, welche die Vorformlinge sterilisiert. Dabei kann diese Sterilisationseinrichtung 4 eine Vielzahl von Strahlungsquellen aufweisen (nicht gezeigt), welche die Vorformlinge 10 insbesondere mittels Elektronenstrahlung sterilisieren. Vorzugsweise sind dabei die einzelnen Strahlungsquellen stationär gehalten und die Behältnisse werden diesen gegenüber bewegt. Die einzelnen Strahlungsquellen sind bevorzugt unabhängig voneinander steuerbar.

Nach Durchlaufen der Umformungseinrichtung 6 gelangen die erzeugten Behältnisse 20 über eine weitere in ihrer Gesamtheit mit 12 bezeichnete zweite Transporteinrichtung in Richtung der Befüllungseinrichtung 40. Dabei kann auch diese zweite Transporteinrichtung 12 eine Vielzahl von Tafeleinheiten aufweisen wie beispielsweise ein Transportrad 48, eine Transportkette 32 und einen Transportstern. Es wäre jedoch auch denkbar, dass die erste Transporteinrichtung 2 und die zweite Transproteinrichtung 12 einteilig, beispielsweise als durchgehend zusammenhängende Transportkette ausgebildet sind.

Das Bezugszeichen 8 bezieht sich auf eine zweite Sterilisationseinrichtung, welche die Behältnisse 20 sterilisiert um beispielsweise neu aufgetretene Verkeimungen zu entfernen. Diese zweite Sterilisationseinrichtung 8 ist dabei bevorzugt unmittelbar nach der Transporteinrichtung 48 angeordnet.

Bei der zweiten Sterilisationseinrichtung 8 handelt es sich bevorzugt um eine mit UV-Strahlung arbeitende Sterilisationseinrichtung. Ausgehend von dieser zweiten Sterilisationseinrichtung 8 gelangen die Behältnisse 20 in Fig. 1 zu der Befüllungseinrichtung und werden damit unmittelbar nach der Sterilisation durch die zweite Sterilisationseinrichtung 8 mit einer Flüssigkeit und insbesondere einem Getränk befüllt. Die Befüllungseinrichtung 40 weist dabei ein Transportrad 41 auf. Anstelle oder neben der Befüllungseinrichtung 40 könnte jedoch auch ein Rinser vorgesehen sein.

Das Bezugszeichen 26 bezieht sich auf ein Gehäuse, welches hier sowohl die Befüllungseinrichtung 40 als auch die beiden Sterilisationseinrichtungen und die Heizeinrichtung sowie die Umformungseinrichtung 6 umgibt. Innerhalb dieses Gehäuses 26 ist eine sterile Umgebung 50 angeordnet.

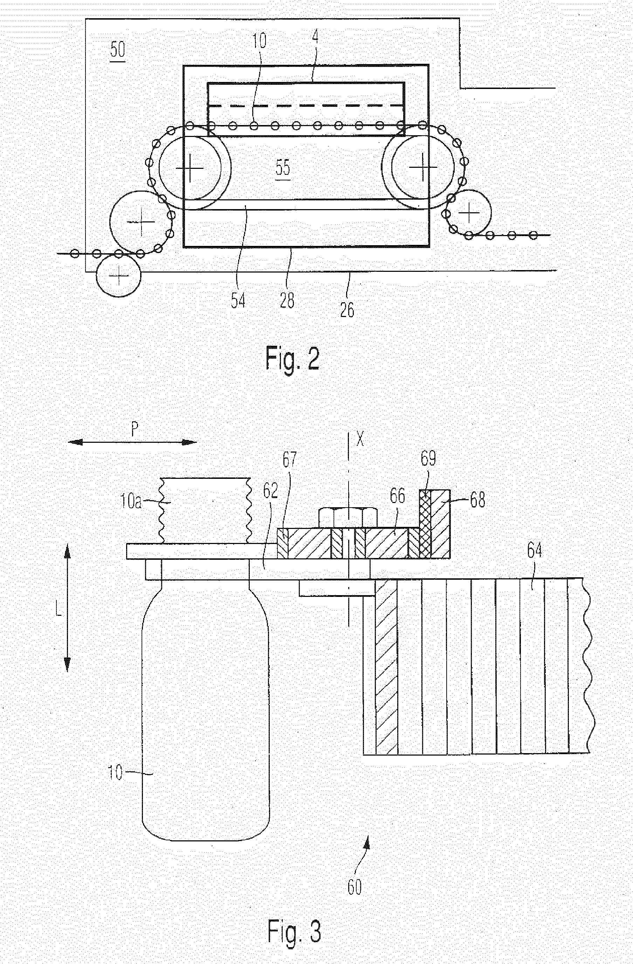

Fig. 2 zeigt eine teilweise Darstellung aus Fig. 1, genauer gesagt den Bereich der Vorrichtung 1, in dem die erste Sterilisationseinrichtung 4 angeordnet ist. Diese Sterilisationseinrichtung 4 kann dabei ebenfalls eine Transportkette 54 mit einer Vielzahl von Greifelementen aufweisen, welche die Behältnisse greifen. Bevorzugt sind diese Greifelemente derart gestaltet, dass sie eine Rotation der Vorformlinge 10 um deren eigene Längsachse erlauben, um auf diese Weise die Sterilisationswirkung zu verbessern.

Das Bezugszeichen 28 bezieht sich auf ein zweites Gehäuse, innerhalb dessen die Sterilisationseinrichtung 4 angeordnet ist. Dieses Gehäuse 28 ist wiederum vollständig innerhalb des oben erwähnten ersten Gehäuses 26 angeordnet. Innerhalb des zweiten Gehäuses 28 befindet sich ein zweiter Sterilraum 55, der insbesondere unter einem höheren Reinheitsgrad gehalten ist als der erste Sterilisationsraum 50.

Fig. 3 zeigt eine schematische Darstellung eines Greifelements 60 welches mit einer Greifklammer einen Vorformling 10 trägt. Dabei ist es möglich, dass dieses Greifelement eine Antriebseinrichtung 66 aufweist, welche eine Drehung des Vorformlings 10 um seine eigene Achse bewirkt. Das Bezugszeichen 64 bezieht sich auf einen Träger, der wiederum die Greifeinrichtung 62 hält. Es wäre jedoch auch möglich, dass mit Hilfe dieses Trägers 64 während des Transports der Vorformlinge noch weitere Bewegungen erzeugt werden wie beispielsweise in Fig. 3 eine Bewegung entlang des Doppelpfeils P. Umgekehrt wäre es jedoch auch möglich, dass die (nicht gezeigten) Strahlungseinrichtungen der ersten Sterilisationseinrichtung 4 sich beispielsweise in der Richtung P gegenüber den Vorformlingen 10 bewegen. Auch wäre es, wie oben gesagt, möglich, dass die entsprechenden Strahlungsquellen in einer Längsrichtung L des Vorformlings 10 oder auch der Richtung P bewegen.

Bei der Antriebseinrichtung 66 handelt es sich hier um ein Rad 66, welches an dem Greifelement 60 angeordnet ist und welches einerseits einen Tragring 10a des Vorformlings kontaktiert und andererseits einen stationären Rand 68, der beispielsweise in einem Bereich der ersten Sterilisationseinrichtung angeordnet sein kann. Bei einer Bewegung des Greifelements 60 in der Transporteinrichtung wird das Rad 66 relativ gegenüber dem Rand bewegt und dreht sich auf diese Weise. Dadurch wird auch der Vorformling über seinen Tragring oder allgemein denjenigen Bereich des Vorformlings, der von dem Rad 66 kontaktiert wird, gedreht.

Es wird darauf hingewiesen, dass das in Fig. 3 dargestellte Greifelement nicht nur zum Drehen von Vorformlingen 10, sondern auch zum Drehen der Behältnisse 20 verwendet werden kann. Vorzugsweise kontaktiert daher das Rad 66 einen Bereich der Mündung des Vorformlings bzw. Kunststoffbehältnisses 20, da dieser Bereich während des Blasvorgangs in der Regel unverändert bleibt. Das Rad 66 ist dabei um eine Achse X drehbar gelagert. Weiterhin kann das Rad 66 mit einem Gummiüberzug 67 oder dergleichen versehen sein, um die Reibung mit dem Tragring 10a des Behältnisses zu erhöhen. Auch der Rand 68 kann einen entsprechenden Gummiüberzug 69 aufweisen.

Die in Fig. 3 dargestellte Einrichtung erlaubt eine mechanisch sehr einfache Drehung der Behältnisse, wobei auch diejenigen Bereiche, in denen eine Drehung stattfindet, einfach bestimmt werden können, in dem entweder ein Rand 68 vorgesehen ist oder nicht. Auch wäre es möglich, den Rand 68 beweglich auszuführen, um ihn insbesondere von dem Rad wegbewegen zu können, um auf diese Weise die Drehung des Kunststoffbehältnisses ab- oder zuschalten zu können.

Die Greifeinrichtung bzw. Greifklammer 62 ist dabei derart ausgelegt, dass sie eine Drehung des Kunststoffvorformlings 10 oder Behältnisses 20 erlaubt, ohne dass dieses nach unten herausfallen kann. So kann beispielsweise die Greifklammer 62 an ihrer dem Kunststoffvorformling oder dem Kunststoffbehältnis zugewandten Oberfläche Lagerelemente wie Rollen aufweisen.

Sämtliche in den Anmeldungsunterlagen offenbarten Merkmale werden als erfindungswesentlich beansprucht, sofern sie einzeln oder in Kombination gegenüber dem Stand der Technik neu sind.

### Bezugszeichenliste

- 1: Vorrichtung
- 2: erste Transporteinrichtung
- 4: erste Sterilisationseinrichtung
- 5: Heizeinrichtung
- 6: Umformungseinrichtung
- 8: zweite Sterilisationseinrichtung
- 10: Kunststoffvorformling
- 10a: Mündung des Kunststoffvorformlings bzw. Kunststoffbehältnisses
- 11: Transportkette
- 12: zweite Transporteinrichtung
- 16: Blasrad
- 18: Transportrad
- 19: Transportrad
- 20: Kunststoffbehältnis
- 26: erstes Gehäuse
- 28: zweites Gehäuse
- 32: Transportkette
- 40: Befüllungseinrichtung
- 41: Transportrad
- 48: Transportrad
- 50: erster Sterilraum
- 55: zweiter Sterilraum
- 60: Greifelement
- 62: Greifklammer
- 64: Träger
- 66: Antriebseinrichtung, Rad
- 67: Gummiüberzug des Rads 66
- 68: Rand
- 69: Gummiüberzug des Rands 68

- L: Längsrichtung der Behältnisse
- P: Bewegungsrichtung

## Patentansprüche

1. Vorrichtung (1) zum Herstellen von Kunststoffbehältern (20) mit einer Umformungseinrichtung (6), welche Kunststoffvorformlinge (10) zu Kunststoffbehältnissen (20) umformt, mit einer ersten Transporteinrichtung (2), welche die Kunststoffvorformlinge (10) der Umformungseinrichtung (6) zuführt und einer zweiten Transporteinrichtung (12), welche die Kunststoffbehältnisse (20) von der Umformungseinrichtung abführt, **dadurch gekennzeichnet, dass**
die Vorrichtung eine erste Sterilisationseinrichtung (4) zum Sterilisieren der Kunststoffvorformlinge (10) aufweist, welche in einer Transportrichtung der Kunststoffvorformlinge vor der Umformungseinrichtung (6) angeordnet ist und eine zweite Sterilisationseinrichtung (8) zum Sterilisieren der Kunststoffbehältnisse (20), welche in der Transportrichtung der Kunststoffbehältnisse (20) nach der Umformungseinrichtung (6) angeordnet ist, wobei wenigstens ein Sterilisationsvorgang durch die Verwendung von Chemikalien gestützt ist.

2. Vorrichtung (1) nach Anspruch 1,
**dadurch gekennzeichnet, dass**
wenigstens ein Sterilisationsvorgang durch die Verwendung von Wasserstoffperoxid (H₂O₂) gestützt ist.

3. Vorrichtung (1) nach Anspruch 1,
**dadurch gekennzeichnet, dass**
die erste Sterilisationseinrichtung (4) eine Strahlungsquelle aufweist, mittels derer die Kunststoffvorformlinge mit einer Strahlung beaufschlagt werden.

4. Vorrichtung (1) nach wenigstens einem der vorangegangenen Ansprüche,
**dadurch gekennzeichnet, dass**
die zweite Sterilisationseinrichtung (8) eine Strahlungsquelle aufweist, mittels derer die Kunststoffbehältnisse (20) mit einer Strahlung beaufschlagt werden.

5. Vorrichtung (1) nach wenigstens einem der vorangegangenen Ansprüche,
**dadurch gekennzeichnet, dass**
die Vorrichtung eine Dreheinrichtung aufweist, welche die Vorformlinge während der Sterilisation durch die erste Sterilisationseinrichtung (4) sterilisiert.

6. Vorrichtung (1) nach wenigstens einem der vorangegangenen Ansprüche,
**dadurch gekennzeichnet, dass**
wenigstens eine Sterilisationseinrichtung (4,8) wenigstens eine Strahlungsquelle (24) aufweist, deren Bewegung an die Bewegung der Kunststoffvorformlinge (10) oder die Bewegung der Kunststoffbehältnisse (20) gekoppelt ist.

7. Vorrichtung (1) nach wenigstens einem der vorangegangenen Ansprüche,
**dadurch gekennzeichnet, dass**
wenigstens ein Abschnitt wenigstens einer Strahlungsquelle in die Mündung der zu sterilisierenden Kunststoffvorformlinge (10) einführbar ist.

8. Vorrichtung (1) nach wenigstens einem der vorangegangenen Ansprüche,
**dadurch gekennzeichnet, dass**
die Vorrichtung eine Heizeinrichtung (5) aufweist, welche die Kunststoffvorformlinge erwärmt.

9. Vorrichtung (1) nach Anspruch 7,
**dadurch gekennzeichnet, dass**
die erste Sterilisationseinrichtung (4) in der Transportrichtung der Kunststoffvorformlinge (10) vor der Heizeinrichtung (5) angeordnet ist.

10. Vorrichtung (1) nach wenigstens einem der vorangegangenen Ansprüche,
**dadurch gekennzeichnet, dass**
die zweite Sterilisationseinrichtung (8) in der Transportrichtung der Behältnisse (20) unmittelbar nach der Umformungseinrichtung (6) angeordnet ist.

11. Vorrichtung (1) nach wenigstens einem der vorangegangenen Ansprüche,
**dadurch gekennzeichnet, dass**
die Umformungseinrichtung (6), und die Sterilisationseinrichtungen (4, 8) innerhalb eines Gehäuses (26) angeordnet sind, wobei dieses Gehäuse (26) in seinem Inneren einen Sterilraum (50) ausbildet.

12. Vorrichtung (1) nach Anspruch 10,
**dadurch gekennzeichnet, dass**
im Bereich der ersten Sterilisationseinrichtung (4) innerhalb des Gehäuses (26) ein zweites Gehäuse (28) vorgesehen ist, wobei innerhalb dieses zweiten Gehäuses (28) die erste Sterilisationseinrichtung (4) angeordnet ist.

13. Verfahren zum Herstellen von Kunststoffbehältnissen (20) aus Kunststoffvorformlingen (10), wobei einer Umformungseinrichtung (6) Kunsstoffvorformlinge (10) mittels einer ersten Transporteinrichtung (2) zugeführt werden und aus den Kunststoffvorformlingen (10) erzeugte Kunststoffbehältnisse (20) mittels einer zweiten Transporteinrichtung (12) von der Umformungseinrichtung (6) abgeführt werden,
**dadurch gekennzeichnet, dass**
die Kunststoffvorformlinge (10) auf ihrem Transportpfad vor der Umformungseinrichtung (6) und die aus den Kunststoffvorformlingen (10) erzeugten Kunststoffbehältnisse (20) auf ihrem Transportpfad nach der Umformungseinrichtung (6) sterilisiert werden wobei wenigstens ein Sterilisationsvorgang durch die Verwendung von Chemikalien gestützt wird.

14. Verfahren nach Anspruch 13,
**dadurch gekennzeichnet, dass**
wenigstens die erste Sterilisation oder die zweite Sterilisation mittels Strahlung erfolgt.

15. Verfahren nach wenigstens einem der vorangegangenen Ansprüche 13 - 14,
**dadurch gekennzeichnet, dass**
beide Sterilisationsvorgänge durch die Verwendung von Chemikalien erfolgen.
